# EUROPÄISCHE PATENTSCHRIFT

(11) **EP 1 935 352 B1**
(45) Veröffentlichungstag und Bekanntmachung des Hinweises auf die Patenterteilung: **30.09.2009**
(21) Anmeldenummer: 07024698.8
(22) Anmeldetag: 20.12.2007
(51) Int. Cl.: A61B 17/17

(54) **Vorrichtung zum Führen eines Bohrwerkzeugs zum Einbringen einer zweiten Bohrung in einem Knochen**
Device for guiding a drill tool in order to create a second drill hole in a bone
Dispositif de commande d'un outil de forage destiné à l'introduction d'un second trou dans un os

(30) Priorität: 22.12.2006 DE 102006062382
(43) Veröffentlichungstag der Anmeldung: 25.06.2008
(73) Patentinhaber: Karl Storz GmbH & Co. KG, 78532 Tuttlingen (DE)
(72) Erfinder: Berberich, Sascha, 78532 Tuttlingen (DE); Sauer, Michael, 78532 Tuttlingen (DE)
(74) Vertreter: Weller, Wolfgang

(56) Entgegenhaltungen:
- EP-A- 1 642 538
- EP-A- 1 714 619
- DE-A1- 3 312 250
- US-A- 4 722 331
- US-A- 5 688 284
- US-A1- 2004 087 953
- US-A1- 2007 191 852
- US-B1- 6 254 605
- US-B1- 6 994 725

## Beschreibung

Die Erfindung betrifft eine Vorrichtung zum Führen eines Bohrwerkzeuges zum Einbringen einer zweiten Bohrung in einem Knochen, die eine bereits vorhandene erste Bohrung schneidet, mit einem Gestell, an dessen ersten Ende ein stabförmiger Körper angeordnet ist, der in die bereits vorhandene erste Bohrung im Knochen einführbar ist, und an dessen zweitem Ende eine Führungshülse für das Bohrwerkzeug angeordnet ist, deren Längsachse den stabförmigen Körper im Bereich des distalen Endes schneidet, wobei an der Führungshülse eine nach distal vorstehende Fixiervorrichtung vorgesehen ist, die an den Körper des Patienten im Bereich der einzubringenden zweiten Bohrung anlegbar ist.

Eine derartige Vorrichtung ist aus der US-A-5 688 284 bekannt.

Derartige Vorrichtungen finden Einsatz beim Fixieren eines in einem Knochen eingesetzten Sehnenersatzes, meist im Bereich des Kniegelenkes.

Dazu wird mit einem Bohrwerkzeug eine erste Bohrung in den Knochen eingebracht, die sowohl durch den Oberschenkelknochen (Femur) als auch durch den Unterschenkelknochen (Tibia) reicht. In diese erste Bohrung wird ein Sehnenersatz eingeschoben.

Zur Fixierung des Sehneneinsatzes wird bei der eingangs genannten Vorrichtung von der Seite her eine weitere Bohrung eingebracht, die in der ersten Bohrung mündet. In diese Querbohrung wird eine Schraube zur Fixierung des in der ersten Bohrung eingeschobenen Sehnenersatzes eingedreht.

Dazu ist es notwendig, eine weitere zweite Bohrung in dem Oberschenkelknochen zu bewerkstelligen, die so ausgerichtet ist, dass diese die erste Bohrung trifft, diese also schneidet.

Da aus dieser Querrichtung die Lage der ersten Bohrung in dem Knochen für den Operateur nicht ersichtlich ist, wurden Hilfs- oder Zielgeräte geschaffen, um diese sich quer zur ersten Bohrung erstreckende zweite Bohrung einzubringen.

Damit bei dem Einbringen der zweiten Bohrung die Führungshülse fest an der Knochenfläche gehalten werden kann, ist eine nach distal vorstehende Fixiervorrichtung vorgesehen. Diese Fixiervorrichtung besteht bei der eingangs genannten Vorrichtung aus kronenartig vorspringenden Zacken am distalen ringförmigen Ende der Führungshülse. Diese Zacken können etwas in den Knochen eingetrieben werden, so dass ein seitliches Abrutschen der Führungshülse gehindert wird.

Aus der US-A-4 722 331 ist eine orthopädische Werkzeugführungsvorrichtung bekannt, über die eine Bohrung in einen Knochen eingebracht werden kann. Die Führungshülse zum Führen des Bohrwerkzeugs ist an ihrem distalen Ende mit einem umfänglichen Kranz an vorstehenden Zacken versehen, die an den Knochen angelegt bzw. etwas in diesen eingetrieben werden können.

Aus der EP-A-1 714 619 ist eine Vorrichtung zum Einbringen einer Bohrung in einem Knochen bekannt, bei dem an dem Bohrdraht ein Zielelement zum Anlegen an den Außenrand des femuralen Ansatzes an einen hinteren Kreuzband aufweist.

Mit den kronen- oder kranzartig vorstehenden Zacken, die in den Knochen eingetrieben werden können, kann zwar ein seitliches Abrutschen der Bohrhülse verhindert werden, bei stark gewölbten Knochenoberflächen können aber nur einige wenige Zacken in den Knochen eindringen und für die Fixierung sorgen.

Es ist daher Aufgabe der vorliegenden Erfindung, eine Vorrichtung der eingangs genannten Art dahin weitergehend zu entwickeln, dass ein Einbringen und eine zielgerichtete Positionierung der zweiten Bohrung möglich ist, ohne das Knochenmaterial unnötig zu traumatisieren, und wobei eine Anpassung an unebene Knochenflächen möglich ist.

Erfindungsgemäß wird die Aufgabe dadurch gelöst, dass Fixierelemente auf einem Haltekörper angeordnet sind, und dass der Haltekörper mit der Führungshülse über ein Kugelgelenk verbunden ist.

Beim Einbringen der zweiten Bohrung in den Knochen mit der derartig ausgestalteten Vorrichtung wird zuerst der stabförmige Körper in die erste bereits vorhandene Bohrung in den Knochen eingeführt und die Führungshülse wird durch Anlegen der Fixiervorrichtung an den Körper des Patienten im Bereich der anzubringenden Bohrung fixiert. Die Anpresskraft der Fixiervorrichtung wird über das Gestell und den stabförmigen Körper erzeugt, der in der ersten Bohrung steckt. Der Haltekörper, der Fixierelemente trägt, übt die Anpresskraft gleichmäßig aus. Dadurch, dass der Haltekörper mit der Führungshülse über ein Kugelgelenk verbunden ist, erfolgt eine sichere, jedoch gelenkige Verbindung zwischen den beiden Bauelementen. Dadurch können gewünschte Abwinkelstellungen des Haltekörpers und damit der auf dem Haltekörper angeordneten Fixierelemente relativ zur Längsrichtung der Führungshülse auf direktem Weg erzielt werden.

In weiteren Ausgestaltungen der Erfindung ist die Fixiervorrichtung über zumindest drei Fixierstellen an dem Körper des Patienten anlegbar.

Durch Anlegen der Fixiervorrichtung über zumindest drei Fixierstellen an den Körper des Patienten wird für einen stabilen, insbesondere kippsicheren Sitz der Führungshülse an dem Körper des Patienten während des Eingriffs gesorgt.

Durch Vorsehen von drei Fixierelementen hat die Führungshülse bei dem Bewerkstelligen der zweiten Bohrung und beim Einbringen des Querstifts in die zweite Bohrung einen festen Halt, so dass die Führungshülse gegen unerwünschte Bewegungen gesichert ist.

In einer weiteren Ausgestaltung der Erfindung sind die Fixierelemente gleichmäßig um den Umfang des Haltekörpers verteilt.

Diese Maßnahme hat den Vorteil, dass durch gleichmäßige Verteilung der zumindest drei Führungselemente um den Umfang des Haltekörpers erreicht wird, dass die Führungshülse an dem Körper des Patienten im Bereich der einzubringenden Bohrung fest und gleichmäßig fixiert ist, wodurch die Stabilität der Fixierung der Führungshülse während des chirurgischen Eingriffs gesichert ist.

In einer weiteren Ausgestaltung der Erfindung sind die Fixierelemente als radial vorspringende Beine ausgebildet.

Diese Maßnahme hat den Vorteil, dass die Ausbildung der Führungselemente als radial vorspringende Beine zum einen eine konstruktiv besonders einfache Möglichkeit darstellt, die Fixiervorrichtung für die zuvor genannten Funktionen auszubilden. Die auf dem Haltekörper angeordneten radial vorspringenden Beine bilden drei Fixierstellen, die für den Operateur sowohl beim Anlegen als auch während des chirurgischen Eingriffs ständig gut sichtbar sind. Somit kann der Operateur, falls notwendig, das Fixieren an den Fixierungsstellen korrigieren, was zur weiteren Erhöhung der Stabilität der Fixierung der Führungshülse während des chirurgischen Eingriffs beiträgt.

In einer weiteren Ausgestaltung der Erfindung sind Enden der Fixierelemente hakenartig ausgebildet.

Diese Maßnahme hat den Vorteil, dass die hakenartig ausgebildeten Enden der Fixierelemente, sich in die Haut hineinstechen, und für einen stabilen Sitz der Führungshülse während des chirurgischen Eingriffs sorgen.

In einer weiteren Ausgestaltung der Erfindung ist der Haltekörper flexibel.

Diese Maßnahme hat den Vorteil, dass die Fixiervorrichtung sich unebenen Körperflächen anpasst. Dies ist bei Operationen im Kniebereich der Fall.

Beim Anlegen an eine unebene Körperfläche üben die auf dem Haltekörper angeordneten Fixierelemente auf den Haltekörper eine gewisse Kraft aus. Die auf den aus flexiblem Material hergestellten Haltekörper ausgeübte Kraft bewirkt seine Verformung, so dass sich die Fixierelemente der unebenen Körperfläche des Patienten im Bereich der einzubringenden Bohrung anpassen können.

In einer weiteren Ausgestaltung der Erfindung, die alternativ zu der zuvor genannten Ausgestaltung verwendet werden kann, sind die Fixierelemente verstellbar.

Auch bei dieser Ausgestaltung der Erfindung wird auf konstruktiv einfache Bauweise erzielt, dass eine derartig ausgebildete Vorrichtung auf unebenen Körperflächen sicher fixiert werden kann.

In einer weiteren Ausgestaltung der Erfindung ist der Haltekörper als scheibenförmiges Element ausgebildet.

Diese Maßnahme hat den Vorteil, dass ein scheibenförmiges Element eine sehr stabile und einfach herzustellende Ausgestaltung des Haltekörpers darstellt.

In einer weiteren Ausgestaltung der Erfindung weist der Haltekörper eine mittig angeordnete Öffnung auf.

Diese Maßnahme hat den Vorteil, dass durch derartige Anordnung der Öffnung in dem Haltekörper, welche sich in axialer Verlängerung des durch die Führungshülse hindurchgehenden Kanals befindet, ermöglicht wird, dass ein Bohrdraht bzw. ein Bohrwerkzeug durch den Kanal in der Führungshülse und durch die Öffnung in dem Haltekörper durchgeführt werden kann.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: eine perspektivische Seitenansicht einer erfindungsgemäßen Vorrichtung;
- Fig. 2: die Vorrichtung von Fig. 1 in teilweise geschnittener Seitenansicht, wobei in der Führungshülse kein Bohrwerkzeug aufgenommen ist;
- Fig. 3: eine vergrößerte Darstellung der Führungshülse und der von der Führungshülse nach distal vorstehenden Fixiervorrichtung von Fig. 1;
- Fig. 4: eine Situation beim Einbringen einer zweiten Bohrung beim Rekonstruieren eines vorderen Kreuzbands, wobei die gesamte erfindungsgemäße Vorrichtung von Fig. 1 ersichtlich ist;
- Fig. 5: eine der Darstellungen von Fig. 4 vergleichbare Darstellung, wobei das Gestell und der stabförmige Körper abgezogen sind;
- Fig. 6: eine der Darstellungen von Fig. 5 vergleichbare Darstellung, wobei in die erste Bohrung im Knochen ein Sehnenersatz eingeschoben ist und in die zweite Bohrung ein Querstift eingeführt ist.

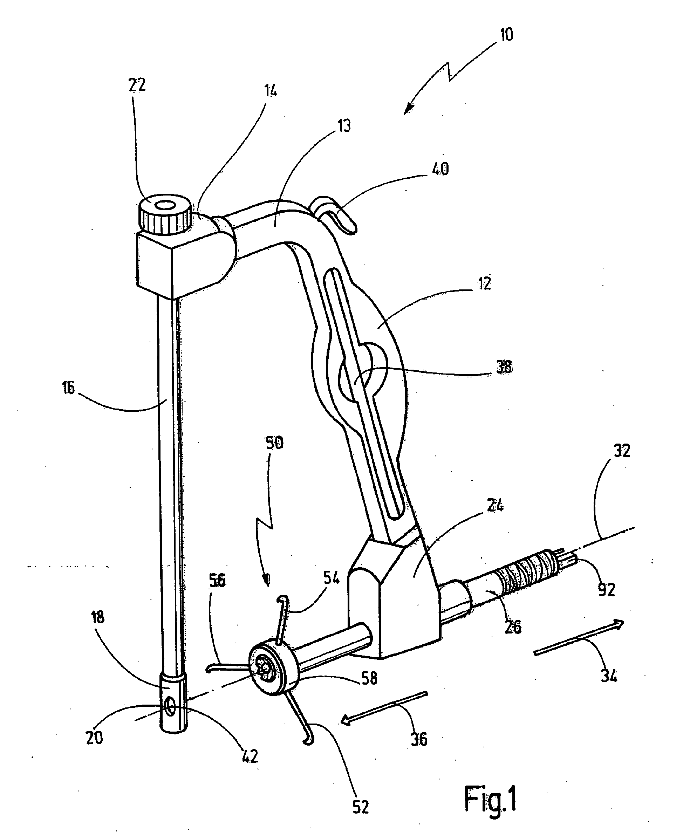

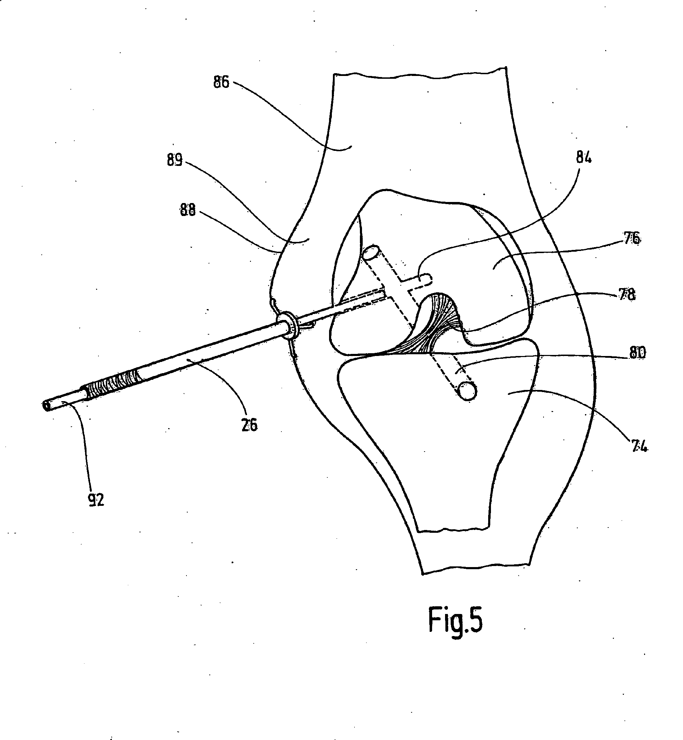

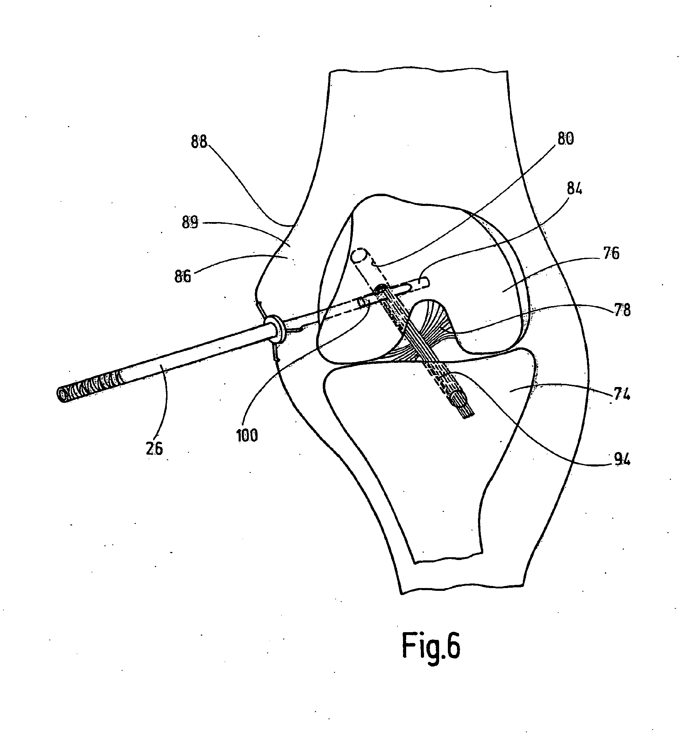

Eine in den Fig. 1 bis 6 dargestellte Vorrichtung zum Führen eines Bohrwerkzeugs zum Einbringen einer zweiten Bohrung in einem Knochen ist in ihrer Gesamtheit mit der Bezugsziffer 10 versehen.

Die Vorrichtung 10 weist ein Gestell 12 auf, das in diesem Ausführungsbeispiel als L-förmiger Bügel 13 ausgebildet ist.

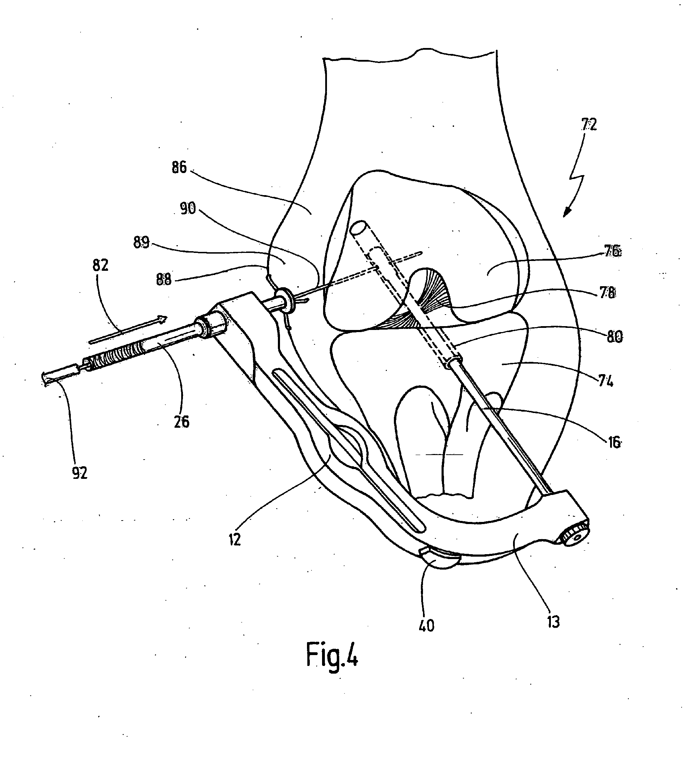

An einem ersten Ende 14 des Gestells 12 ist ein stabförmiger Körper 16 angeordnet, der in eine bereits vorhandene erste Bohrung in einem Knochen einführbar ist, wie das später noch näher in Zusammenhang mit Fig. 4 bis 6 beschrieben wird.

Der stabförmige Körper 16 weist einen kreisförmigen Querschnitt auf.

An einem distalen Ende 18 des stabförmigen Körpers 16 ist eine Öffnung 20 in Form einer hindurchgehenden Querbohrung vorhanden. Der stabförmige Körper 16 ist hohl.

Der stabförmige Körper 16 ist mit dem ersten Ende 14 des Gestells 12 über einer Schraube 22 lösbar verbunden.

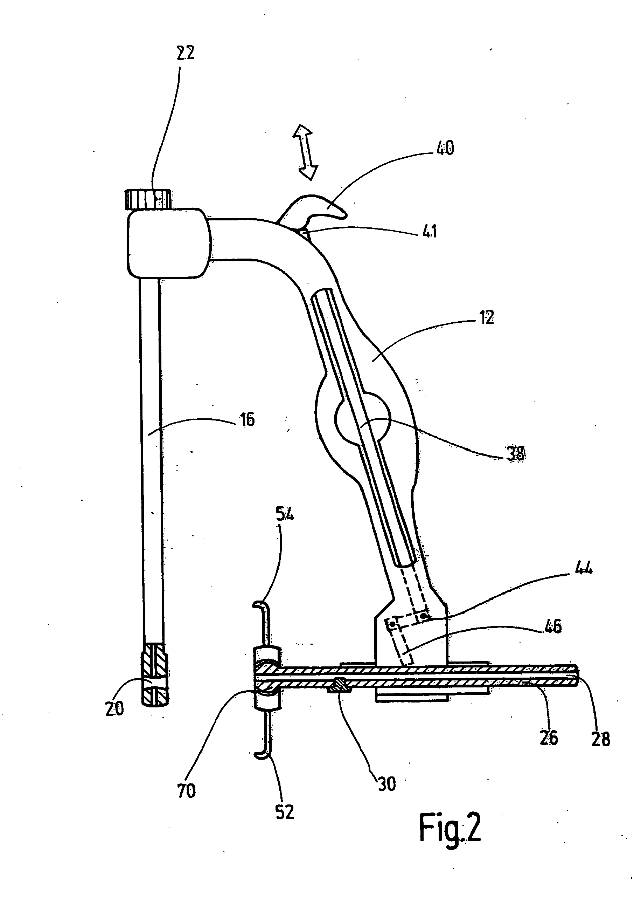

In einem zweiten Ende 24 des Gestells 12 ist eine Führungshülse 26 verschiebbar aufgenommen. Durch die Führungshülse 26 geht ein Kanal 28 hindurch, wie es insbesondere aus der Schnittdarstellung von Fig. 2 ersichtlich ist. In dem Kanal 28 kann ein Bohrwerkzeug 92 aufgenommen werden, wie es aus der Fig. 1 hervorgeht.

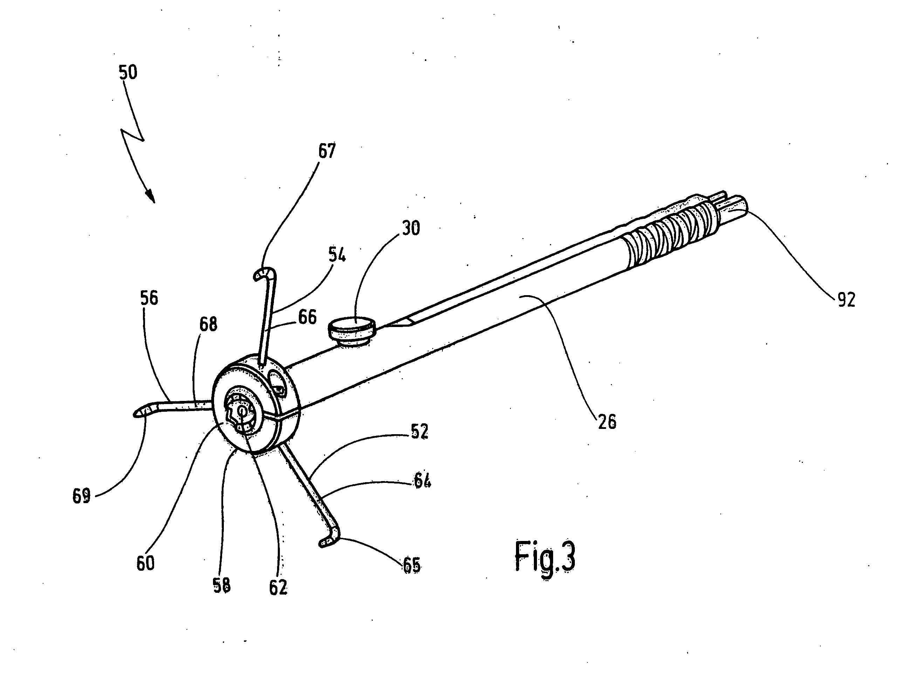

Aus der Darstellung von Fig. 2 und 3 ist ersichtlich, dass die Führungshülse 26 eine Feststellschraube 30 aufweist. Die Feststellschraube 30 dient zum Fixieren eines in dem Kanal 28 der Führungshülse 26 aufgenommenen und hin und her verschiebbaren Bohrdrahts bzw. des Bohrwerkzeugs 92.

Die Führungshülse 26 selbst ist ebenfalls entlang ihrer Längsachse 32 im zweiten Ende 24 hin und her verschiebbar, wie es durch Pfeile 34, 36 in Fig. 1 angezeigt ist.

Eine Klemmvorrichtung dient zum Feststellen der Führungshülse 26 durch Verklemmen.

In dem L-förmigen Bügel 13 des Gestells 12 ist eine bewegliche Klemmstange 38 angeordnet. Diese ist durch einen Hebel 40 betätigbar, der an einer Außenseite des Gestells 12 angeordnet ist und somit für den Operateur einfach betätigbar. Eine Nocke 41 des Hebels 40 ruht auf dem oberen Ende der Klemmstange 38.

Das untere Ende der Klemmstange 38 steht mit einem Schwenkhebel 44 in Verbindung, dessen einer Schenkel 46 mit der Außenseite der Führungshülse 26 in klemmende Verbindung treten kann.

Ein Drücken des Hebels 40 bewirkt ein Anlegen des Schenkels 46 an die Führungshülse 26, wodurch das Verschieben der Führungshülse 26 in die durch den Pfeil 34 angezeigte Richtung gesperrt wird.

Ein Anheben des Hebels 40 gibt den Schwenkhebel 44 und somit die Führungshülse 26 frei.

Aus der Darstellung von Fig. 1 und 2 ist ersichtlich, dass die Führungshülse 26 derart ausgerichtet ist, dass deren Längsachse 32 in Richtung einer Stelle 42 ausgerichtet ist, die in etwa in der Mitte der Öffnung 20 in dem stabförmigen Körper 16 liegt.

Wird in die Führungshülse 26, wie das später noch näher beschrieben wird, ein Führungsdraht bzw. Bohrwerkzeug eingeschoben, wird dies längs der Längsachse 32 zielgerichtet auf die Öffnung 20 an dem distalen Ende 18 des stabförmigen Körpers 16 ausgerichtet.

An der Führungshülse 26 ist eine nach distal vorstehende Fixiervorrichtung 50 angeordnet.

In dem gezeigten Ausführungsbeispiel ist die Fixiervorrichtung 50 an einem distalen Ende der Führungshülse 26 angeordnet.

Die Fixiervorrichtung 50 weist einen Haltekörper 58 auf, auf dem drei Führungselemente 52, 54, 56 angeordnet sind. Der Haltekörper 58 ist in diesem Ausführungsbeispiel als ein ringscheibenförmiges Element 60 ausgebildet. Mittig in dem Element 60 ist eine Öffnung 62 vorhanden, die zur Befestigung des Haltekörpers 58 am distalen Ende der Führungshülse dient.

Die Fixierelemente 52, 54, 56 sind als radial vorspringende Beine 64, 66, 68 ausgebildet. Enden 65, 67, 69 der Fixierelemente 52, 54, 56 sind hakenartig ausgebildet.

Die als Beine 64, 66, 68 ausgebildeten Fixierelemente 52, 54, 56 sind gleichmäßig um den Umfang des Haltekörpers 58 verteilt, d.h. dass die Fixierelemente 52, 54, 56 jeweils um etwa 120° versetzt auf dem Haltekörper 58 angeordnet sind.

Der Haltekörper 58 ist mit der Führungshülse 26 über ein Kugelgelenk 70, wie aus der Schnittdarstellung von Fig. 2 ersichtlich, verbunden. Somit sind unterschiedliche Abwinkelstellungen des Haltekörpers relativ zur Längsrichtung der Führungshülse 26 erreichbar. Die spinnenartigen Beine 64, 66, 68 erlauben durch deren Elastizität eine weitere Feinanpassung an unebene Körperflächen.

Anhand Fig. 4 bis 6 soll die Handhabung der erfindungsgemäßen Vorrichtung 10 kurz erläutert werden.

In Fig. 4 ist ein menschliches Kniegelenk 72 stark schematisiert dargestellt.

Das Kniegelenk 72 ist ein Verbindungsgelenk zwischen der Tibia (Unterschenkelknochen) 74 und dem Femur (Oberschenkelknochen) 76. In Zentrum des menschlichen Knies liegen zwei sich überkreuzende Bänder, die von der Tibia 74 zum Femur 76 verlaufen, nämlich das hintere Kreuzband 78 und das vordere Kreuzband, das hier nicht ersichtlich ist, und das durch ein Implantat ersetzt werden soll.

Bei einer Rekonstruktion des abgerissenen vorderen Kreuzbands, wie in Fig. 4 dargestellt, wird zuerst mit einem chirurgischen Instrument eine erste Bohrung 80 bewerkstelligt, die sich sowohl durch die Tibia 74 als auch in das Femur 76 hineinerstreckt. Die Ausrichtung entspricht etwa der natürlichen Erstreckung des zu ersetzenden vorderen Kreuzbands. Die Bohrung 80 setzt sich meist über einen durchmessergeringen Kanal bis zur Außenseite des Femur 76 fort.

In die erste Bohrung 80 wird der stabförmige Körper 16 der Vorrichtung 10 eingeführt. Dann wird die an dem zweiten Ende 24 des Gestells 12 angeordnete Führungshülse 26 mit der von der Führungshülse 26 nach distal vorstehenden Fixiervorrichtung 50 vom Operateur an einer anatomisch günstigen Stelle zielgerecht positioniert.

Dann wird die Führungshülse 26 mit der Fixiervorrichtung 50 soweit in die durch einen Pfeil 82 angezeigte Richtung vom Operateur verschoben, bis die hakenartig ausgebildeten Enden 65, 67, 69 der Fixierelemente 52, 54, 56 an den Körper 86 des Patienten im Bereich der einzubringenden zweiten Bohrung 84 gelangen.

Durch ein weiteres Verschieben der Führungshülse 26 stechen die hakenartig ausgebildeten Enden 65, 67, 69 der Beine 64, 66, 68 durch die Haut 88 in das Gewebe 89 hinein, wodurch die Führungshülse 26 in der vom Operateur gewählten Position zum Einbringen der zweiten Bohrung 84 fixiert wird.

Die Fixiervorrichtung 50 kann sich Dank des Kugelgelenks 70 an Unebenheiten des Körpers des Patienten, der im Bereich des Kniegelenks uneben ist, anpassen. Die Ausrichtung der Führungshülse 26 ändert sich dabei nicht mehr. Durch Feststellen der Klemmvorrichtung verbleibt die Führungshülse 26 in dieser Längsverschiebeposition. Der in der ersten Bohrung steckende stabförmige Körper 16 und der L-förmige Bügel 13 üben den notwendigen Anpressdruck dadurch aus, dass der L-förmige Bügel 13 etwas vom stabförmigen Körper weggespreizt wird. Somit kann die zweite Bohrung 84 mit großer Präzision bewerkstelligt werden.

Es ist ebenfalls vorgesehen, um die Anpassung an die Unebenheiten des Körpers 86 des Patienten noch zu verbessern, entweder den Haltekörper 58 aus einem nachgiebigen Material herzustellen oder als Alternative dazu die Beine 64, 66, 68 verstellbar auszubilden.

Nachdem die Führungshülse 26 mittels der Fixiervorrichtung 50 an dem Körper 86 des Patienten in der gewünschten Position fixiert ist, wird durch den Kanal 28 der Führungshülse 26 zuerst ein Bohrdraht 90 durch die Haut 88 und das Gewebe 89 in den Femur 76 eingetrieben, und zwar so weit, dass der Bohrdraht 90 durch die Öffnung 20 in dem sich in der ersten Bohrung 80 befindlichen stabförmigen Körper 16 hindurchgeht, so wie in Fig. 4 dargestellt ist.

Dann wird durch den Kanal 28 ein Hohlbohrer 92, über den Bohrdraht 90 hinweg in den Femur 76 eingetrieben, der dabei exakt auf die Öffnung 20 in dem stabförmigen Körper 16 ausgerichtet ist.

Nachdem die zweite Bohrung 84 bewerkstelligt worden ist, wird die klemmende Verbindung zwischen L-förmigem Bügel 13 und Führungshülse 26 gelöst und der stabförmige Körper 16 samt dem Gestell 12 wird abgezogen. Die Führungshülse 26 verbleibt somit im Hohlbohrer 92 am bzw. im Körper. Durch die Fixiervorrichtung 50 verbleibt die Führungshülse 26 am Körper von diesem abstehend. Der Hohlbohrer 92 wird so weit zurückgezogen, dass die erste Bohrung 80 frei liegt.

Eine präparierte Sehne wird als ein Kreuzbandersatz 94 mit Hilfe eines Zugfadens in die erste Bohrung 80 als Doppelschlinge eingezogen. Der Zugfaden 96 wird dann an der Außenseite des Körpers 86 durch einen Fixierknopf 98 fixiert.

Jetzt wird das Bohrwerkzeug 92 aus der Führungshülse 26 entfernt und durch den in der Führungshülse 26 vorhandenen Kanal 28 wird in die zweite Bohrung 84 ein Querstift 100 eingeführt. Mittels des Querstifts 100 wird der Kreuzbandersatz 94 gegen Abziehen aus der ersten Bohrung 80 fixiert.

In der ersten Bohrung 80 werden die Enden des Kreuzbandersatzes 94 z.B. über einen hier nicht dargestellten Knochendübel oder einen weiteren Fixierknopf fixiert.

## Patentansprüche

1. Vorrichtung zum Führen eines Bohrwerkzeugs (92) zum Einbringen einer zweiten Bohrung (84) in einem Knochen, die eine bereits vorhandene erste Bohrung (80) schneidet, mit einem Gestell (12), an dessen erstem Ende (14) ein stabförmiger Körper (16) angeordnet ist, der in die bereits vorhandene erste Bohrung (80) im Knochen einführbar ist, und an dessen zweitem Ende (24) eine Führungshülse (26) für das Bohrwerkzeug (92) angeordnet ist, deren Längsachse (32) den stabförmigen Körper (16) im Bereich des distalen Endes (18) schneidet, wobei an der Führungshülse (26) eine nach distal vorstehende Fixiervorrichtung (50) vorgesehen ist, die an den Körper (86) des Patienten im Bereich der einzubringenden zweiten Bohrung (84) anlegbar ist, **dadurch gekennzeichnet, dass** Fixierelemente (52, 54, 56) auf einem Haltekörper (58) angeordnet sind, und dass der Haltekörper (58) mit der Führungshülse (26) über ein Kugelgelenk (70) verbunden ist.

2. Vorrichtung nach Anspruch 1, **dadurch gekennzeichnet, dass** die Fixiervorrichtung (50) über zumindest drei Fixierstellen an dem Körper (86) des Patienten anlegbar ist.

3. Vorrichtung nach Anspruch 1 oder 2, **dadurch gekennzeichnet, dass** die Fixiervorrichtung (50) zumindest drei Fixierelemente (52, 54, 56) aufweist.

4. Vorrichtung nach Anspruch 1, **dadurch gekennzeichnet, dass** die Fixierelemente (52, 54, 56) gleichmäßig um den Umfang des Haltekörpers (58) verteilt sind.

5. Vorrichtung nach Anspruch 1, **dadurch gekennzeichnet, dass** die Fixierelemente (52, 54, 56) als radial vorspringende Beine (64, 66, 68) ausgebildet sind.

6. Vorrichtung nach einem der Ansprüche 1 bis 5, **dadurch gekennzeichnet, dass** Enden (65, 67, 69) der Fixierelemente (52, 54, 56) hakenartig ausgebildet sind.

7. Vorrichtung nach einem der Ansprüche 1 bis 6, **dadurch gekennzeichnet, dass** der Haltekörper (58) flexibel ist.

8. Vorrichtung nach einem der Ansprüche 1 bis 7, **dadurch gekennzeichnet, dass** die Fixierelemente (52, 54, 56) verstellbar sind.

9. Vorrichtung nach einem der Ansprüche 1 bis 8, **dadurch gekennzeichnet, dass** der Haltekörper (58) als scheibenförmiges Element (60) ausgebildet ist.

10. Vorrichtung nach einem der Ansprüche 1 bis 9, **dadurch gekennzeichnet, dass** der Haltekörper (58) eine mittig angeordnete Öffnung (62) aufweist.

## Claims

1. Device for guiding a drilling tool (92) for forming a second bore (84) in a bone, which second bore (84) intersects an already existing first bore (80), with a frame (12) at whose first end (14) a rod-shaped body (16) is arranged which can be inserted into the already existing first bore (80) in the bone, and at whose second end (24) a guide sleeve (26) for the drilling tool (92) is arranged, with the longitudinal axis (32) of the guide sleeve (26) intersecting the rod-shaped body (16) in the area of its distal end (18), wherein on the guide sleeve (26) a distally protruding fixing device (50) is provided, which can be placed on the body (86) of the patient in the area where the second bore (84) is to be formed, **characterized in that** fixing elements (52, 54, 56) are arranged on a holder body (58), and **in that** the holder body (58) is connected to the guide sleeve (26) via a ball joint (70).

2. Device of claim 1, **characterized in that** the fixing device (50) can be placed on the body (86) of the patient over at least three fixing sites.

3. Device of claims 1 or 2, **characterized in that** the fixing device (50) has at least three fixing elements (52, 54, 56).

4. Device of claim 1, **characterized in that** the fixing elements (52, 54, 56) are distributed uniformly about the circumference of the holder body (58).

5. Device of claim 1, **characterized in that** the fixing elements (52, 54, 56) are designed as radially protruding legs (64, 66, 68).

6. Device of anyone of claims 1 through 5, **characterized in that** ends (65, 67, 69) of the fixing elements (52, 54, 56) are hook-shaped.

7. Device of anyone of claims 1 through 6, **characterized in that** the holder body (58) is flexible.

8. Device of anyone of claims 1 through 7, **characterized in that** the fixing elements (52, 54, 56) are adjustable.

9. Device of anyone of claims 1 through 8, **characterized in that** the holder body (58) is designed as a disk-shaped element (60).

10. Device of anyone of claims 1 through 9, **characterized in that** the holder body (58) has a centrally arranged opening (62).

## Revendications

1. Dispositif de guidage d'un outil de forage (92) pour pratiquer, dans un os, un second perçage (84) croisant un premier perçage (80) déjà présent, comportant un châssis (12) à la première extrémité (14) duquel se trouve un corps (16) en forme de tige pouvant être introduit dans le premier perçage (80) déjà présent dans l'os, et à la seconde extrémité (24) duquel est placée une douille (26) de guidage de l'outil de forage (92), douille dont l'axe longitudinal (32) croise ledit corps (16) en forme de tige dans la région de l'extrémité distale (18), un système (50) de verrouillage à demeure, prévu sur la douille de guidage (26) et saillant dans la direction distale, pouvant être mis en applique contre le corps (86) du patient, dans la région du second perçage (84) à façonner, **caractérisé par le fait que** des éléments (52, 54, 56) de consignation à demeure sont disposés sur un corps de retenue (58) ; et **par le fait que** ledit corps de retenue (58) est relié à la douille de guidage (26) par l'intermédiaire d'une articulation sphérique (70).

2. Dispositif selon la revendication 1, **caractérisé par le fait que** le système (50) de verrouillage à demeure peut être mis en applique contre le corps (86) du patient par l'intermédiaire d'au moins trois zones de consignation à demeure.

3. Dispositif selon la revendication 1 ou 2, **caractérisé par le fait que** le système (50) de verrouillage à demeure compte au moins trois éléments (52, 54, 56) de consignation à demeure.

4. Dispositif selon la revendication 1, **caractérisé par le fait que** les éléments (52, 54, 56) de consignation à demeure sont uniformément répartis autour de la périphérie du corps de retenue (58).

5. Dispositif selon la revendication 1, **caractérisé par le fait que** les éléments (52, 54, 56) de consignation à demeure sont réalisés sous la forme de jambages (64, 66, 68) faisant saillie radialement.

6. Dispositif selon l'une des revendications 1 à 5, **caractérisé par le fait que** des extrémités (65, 67, 69) des éléments (52, 54, 56) de consignation à demeure sont réalisées à la manière de crochets.

7. Dispositif selon l'une des revendications 1 à 6, **caractérisé par le fait que** le corps de retenue (58) est flexible.

8. Dispositif selon l'une des revendications 1 à 7, **caractérisé par le fait que** les éléments (52, 54, 56) de consignation à demeure sont réglables.

9. Dispositif selon l'une des revendications 1 à 8, **caractérisé par le fait que** le corps de retenue (58) est réalisé sous la forme d'un élément discoïdal (60).

10. Dispositif selon l'une des revendications 1 à 9, **caractérisé par le fait que** le corps de retenue (58) présente un orifice (62) occupant une position centrale.
